Europäisches Patentamt

European Patent Office

Office européen des brevets

(19)

(11) Veröffentlichungsnummer: **0 267 935**
**B1**

(12)     **EUROPÄISCHE PATENTSCHRIFT**

(45) Veröffentlichungstag der Patentschrift: **09.05.90**

(21) Anmeldenummer: **87903309.0**

(22) Anmeldetag: **21.05.87**

(86) Internationale Anmeldenummer:
**PCT/EP87/00266**

(87) Internationale Veröffentlichungsnummer:
**WO 87/07151 03.12.87 Gazette 87/27**

(51) Int. Cl.⁵: **A 61 L 2/26**

(54) **VENTIL FÜR STERILISIERBEHÄLTER UND VERFAHREN ZUM STEUEREN DES VENTILS.**

(30) Priorität: **22.05.86 DE 3617153**

(43) Veröffentlichungstag der Anmeldung:
**25.05.88 Patentblatt 88/21**

(45) Bekanntmachung des Hinweises auf die
Patenterteilung:
**09.05.90 Patentblatt 90/19**

(84) Benannte Vertragsstaaten:
**AT BE CH DE FR GB IT LI NL SE**

(56) Entgegenhaltungen:
**US-A-4 228 914**
**US-A-4 457 327**

(73) Patentinhaber: **Wagner GmbH**
**Schulstrasse 16a**
**D-8000 München 19 (DE)**

(72) Erfinder: **LORENZ, Jürgen, W.**
**Heiterwangerstr. 20**
**D-8000 München 70 (DE)**

(74) Vertreter: **Koch, Günther, Dipl.-Ing. et al**
**Postfach 920**
**D-8000 München 33 (DE)**

Courier Press, Leamington Spa, England.

EP 0 267 935 B1

**Beschreibung**

Die Erfindung bezieht sich auf ein Ventilsteuerverfahren der im Oberbegriff des Patentanspruchs 1 angegebenen Gattung und auf ein ventil gemäß dem Oberbegriff des Patentanspruchs 5 zur Durchführung des Steuerverfahrens.

Sterilisierbehälter dienen zur Aufnahme klinischen Sterilgutes, das einer Dampfsterilisation unterworfen werden muß. Diese Sterilisation findet in Sterilisatoren statt, die entweder nach dem Gravitationsverfahren (Strömungsverfahren) arbeiten oder in modernen Anlagen zunehmend nach einem Vakuumverfahren, bei dem zunächst eine oder mehrere Vakuum-Phasen vorgesehen sind, um die Luft aus dem Behälter abzusaugen, wonach das Behälterinnere einer Dampfatmosphäre unter erhöhtem Druck und erhöhter Temperatur ausgesetzt wird, wobei das Sterilisiergut einer Sterilisation unterworfen wird. Dabei ist es vorteilhaft, das sich bildende Kondensat abzuleiten, damit nach der Sterilisation das Gut möglichst trocken zur Verfügung steht. Nach der Dampfsterilisationszeit wird der Behälter einer weiteren Vakuum-Behandlung unterzogen, um den Sterilisationsdampf mit etwas verbliebenem Kondensat aus dem Behälter abzuziehen.

Zur Ermöglichung des Medienaustausches sind die Sterilisierbehälter entweder mit Filtern ausgestattet, die einen Medienaustausch zulassen, aber eine Rekontaminierung verhindern, oder es werden in der Behälterwand ventile angebracht, die nach vollendetem Druckaustausch schließen. Als Einström- bzw. Vakuum-Ventile werden häufig Rückschlagventile gemäß der DE-A-12 17 551 oder ein Doppelventil gemäß der DE-A-12 17 550 benutzt. Als Kondensatableitventil haben sich Bimetallventile eingeführt, die gegenüber reinen Rückschlagventilen den Vorteil haben, daß eine Öffnung des Ventils zwecks Kondensatableitung während der gesamten Sterilisierstufe erfolgen kann.

Ein gattungsgemäß ausgebildetes Ventil, welches sämtliche Funktionen durchführen kann, ist aus der US-A-42 28 914 bekannt. Hierbei wird die auf den Ventilkörper einwirkende Gaskammer während der Dampfeinströmphase mit Dampf angefüllt und am Ende der Dampfeinströmphase, d. h. zu Beginn der Sterilisationszeit wird über einen Schrumpfschlauch die Einströmöffnung nach der Gaskammer verschlossen, so daß bei der anschließenden Druckverminderung eine Ausdehnung der Gaskammer ein Schließen des Ventiles bewirken kann. Diese Ausbildung setzt voraus, daß nach jedem Sterilisiervorgang der Schrumpfschlauch ausgewechselt werden muß, um die Abdichtungsfunktion sicherzustellen. Die Abdichtungsfunktion kann dann nicht ordnungsgemäß erfolgen, wenn der Schrumpfschlauch keine sichere Abdichtung herstellt. Erfolgt an der Einströmöffnung ein Leckstrom, dann ist die Abdichtung in Frage gestellt, weil die für den Schließdruck erforderliche Druckdifferenz nicht zustande kommen kann.

Der Erfindung liegt die Aufgabe zugrunde, ein betriebssicher arbeitendes Ventilsystem zuschaffen, welches automatisch ohne Ergänzung und Einstellung von Teilen zuverlässig arbeitet und auf vielfache Weise Anwendung finden kann.

Gelöst wird die gestellte Aufgabe durch die im Kennzeichnungsteil des Patentanspruchs 1 bzw. des Patentanspruchs 5 angegebenen Merkmale.

Dadurch, daß das Gasvolumen ständig in der Kammer verbleibt und nicht, wie beim Stande der Technik, bei jedem Sterilisiervorgang ergänzt werden muß, kann eine zuverlässige und leckstromfreie Abdichtung auf einfache Weise erreicht werden, wobei dieses Gasvolumen unter einem definierten Druck bei vorbestimmter Temperatur, insbesondere unter atmosphärischem Druck, eingebracht werden kann und eine zuverlässige Arbeitsweise hierdurch gewährleistet ist. Zweckmäßigerweise wird der Differenzdruck-Ventilfunktion eine Federvorspannung überlagert, wodurch sich die Ventilöffnungs- bzw. -schließvorgänge gegenüber der atmosphärischen Linie etwas verschieben. Dies ist jedoch zulässig und erwünscht, wobei die temperaturbedingten Druckunterschiede innerhalb der Gaskammer hinsichtlich der angestrebten Funktion ausreichend kompensiert werden können.

Der besondere Unterschied der Erfindung gegenüber gebräuchlichen Rückschlagventilen besteht darin, daß hierbei als Steuerkraft nicht die Druckdifferenz, die auf die Ventilscheibe wirkt, dient, sondern eine Kraft, die aus der Kompression oder Elongation eines Druckaufnehmers resultiert (Rollmembrane, Membrane, Faltenschlauch, Wellrohr, Druckzylinder, Barometerdose usw.), die über Steuerstangen, Steuerscheiben, Hebel, oder aber (bevorzugt) direkt den ventilhub bewirkt. Dabei spielt also der in der Sterilisierkammer vorliegende Druck für den Öffnungs- bzw. Schließzustand des ventils die entscheidende Rolle und nicht das (Nicht-) Vorhandensein einer Druckdifferenz am ventil selbst. Die vom Druckaufnehmer erzeugte Steuerkraft kann noch verstärkt werden, indem ein Teil der Gasdruckfeder oder diese auch vollständig durch eine Flüssigkeit (bevorzugt Wasser) ergänzt (ersetzt) wird, deren Siedepunkt auf den (meistens um 100°C liegenden) erwunschten Schaltpunkt eingestellt ist. Hierbei ergibt sich dann eine Kombination aus druck und temperaturabhängiger Steuerung.

Das erfindungsgemäße Ventil kann als Einströmventil zur Steuerung des Einströmgasstrahls ausgebildet sein oder als Vakuumventil zum Absaugen des Dampfes, und schließlich kann das Ventil auch als Kondensat-Ableitventil Anwendung finden.

Durch entsprechende Ventilausbildung und Anordnung kann erreicht werden, daß der Behälter nach der letzten Vakuumphase nicht wieder vollkommen belüftet wird, so daß der Behälterinhalt bis zur Bereitstellung im Operationssaal einem Vakuum unterworfen wird, wobei das Eindringen der Luft dann beim Öffnen des Behälters erkennbar macht, daß dieses Vakuum bis zuletzt vorhanden war.

Weitere zweckmäßige Ausgestaltungen der Erfindung ergeben sich aus den Unteransprüchen.

Nachstehend werden Ausführungsbeispiele der Erfindung anhand der Zeichnung beschrieben. In der Zeichnung zeigen:

Fig. 1 eine auseinandergezogene perspektivische Darstellung eines Sterilisierbehälters mit erfindungsgemäß ausgebildeten Ventilen;

Fig. 2 einen teilweise abgebrochenen Längsschnitt des Sterilisierbehälters gemäß Fig. 1;

Fig. 3 verschiedene Ausführungsbeispiele von bis Ventilen, die in Verbindung mit einem

Fig. 11 Sterilisierbehälter gemäß Fig. 1 und 2 einsetzbar sind;

Fig. 12 ein Diagramm, das einen möglichen zeitabhängigen Druckverlauf während eines Sterilisierzyklus zusammen mit der Ventilfunktion der einzelnen Ventile zeigt;

Fig. 13 eine schematische Ansicht eines gemäß der Erfindung ausgebildeten Doppelventils.

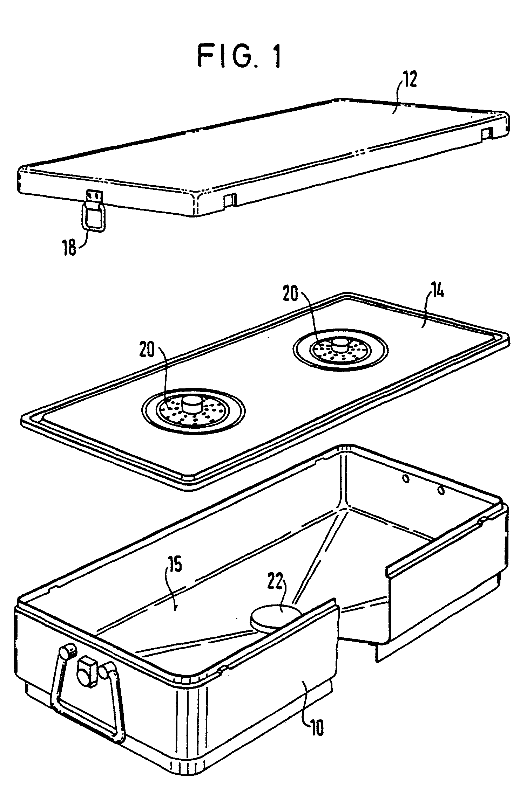

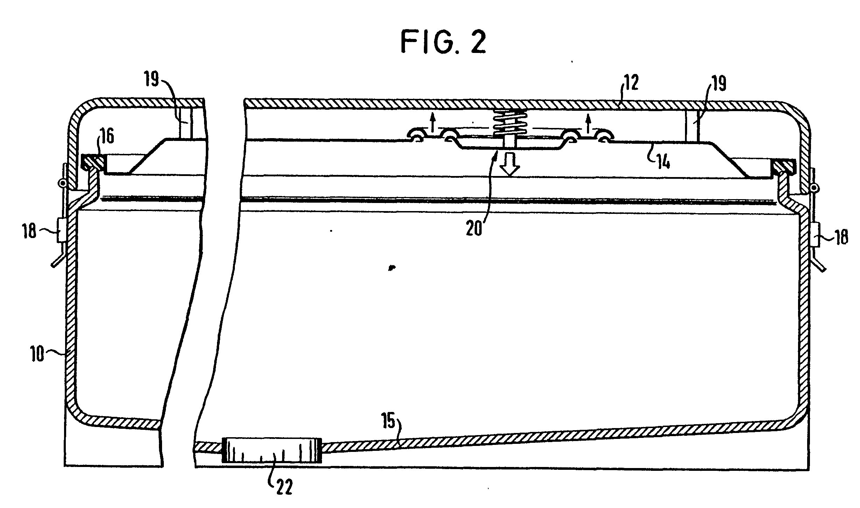

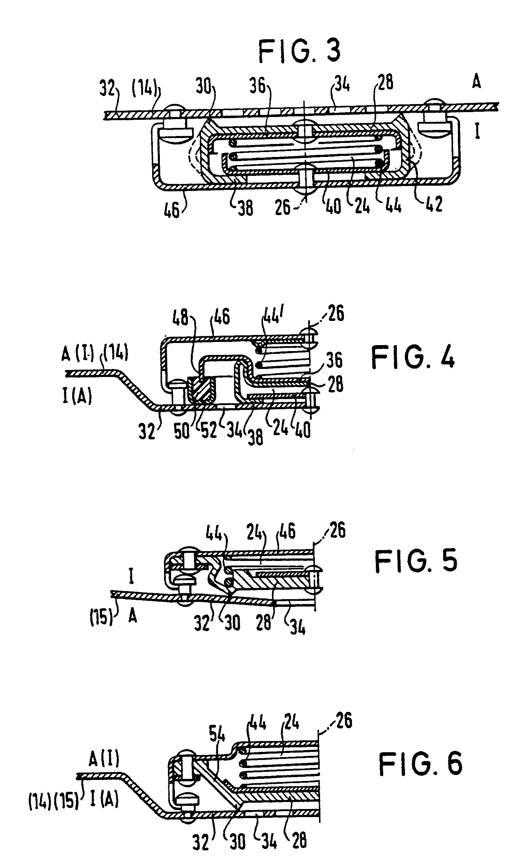

Fig. 1 zeigt einen Sterilisierbehälter der in der DE-PS 31 46 349 beschriebenen Bauart. Dieser Sterilisierbehälter weist einen Behälterunterteil 10, einen Behälterdeckel 12 und einen von letzterem getragenen Zwischendeckel 14 auf. Der Zwischendekkel 14 ist gegenüber dem oberen Rand des Behälterunterteils 10 über einen Dichtungsring 16 abgedichtet. Der Deckel 12 ist mit dem Behälterunterteil 10 über Spannverschlüsse 18 lösbar befestigt, wodurch über die den Zwischendeckel 14 tragenden Stützen 19 der Zwischendeckel 14 dichtend gegen den Unterteil 10 verspannt wird. Der Zwischendeckel 14 ist mit Ventilen 20 ausgestattet, die in Fig. 1 und 2 nur schematisch angedeutet sind. Der Boden des Behälterunterteils 10 fällt, wie aus Fig. 1 ersichtlich, von allen Seiten nach der Mitte ab, wo ein weiteres, in Fig. 1 und 2 nur schematisch angedeutetes Ventil 22 angeordnet ist, welches als Kondensatableiter dienen kann. Diese ventile 20 und 22 bilden den Gegenstand der Erfindung und sind in verschiedenen erfindungsgemäßen Ausführungsbeispielen in den Fig. 3 bis 11 dargestellt. Diese Ventile können bei Sterilisierbehältern der verschiedensten Bauart angewendet werden und ihre Anwendung ist nicht auf das in Fig. 1 und 2 dargestellte Ausführungsbeispiel beschränkt. Insbesondere können die ventile 20 in einem dichtend auf dem Behälterunterteil aufgesetzten Behälterdeckel oder auch im Behälterunterteil angebracht sein.

Allen Ausführungsbeispielen gemeinsam ist eine allseitig abgedichtete Gaskammer 24, die wenigstens teilweise von elastischen Wänden begrenzt ist und in die ein vorbestimmtes Gasvolumen unter vorbestimmtem Druck vorzugsweise trockene Luft unter atmosphärischem Druck eingeschlossen ist. Alle ventile sind rotationssymmetrisch bezüglich einer Mittelachse 26 angeordnet. Bei allen Ausführungsbeispielen (außer bei dem Ausführungsbeispiel nach Fig. 4) trägt eine der gegenüberliegenden kreisrunden Kammerwände 28 einen Ventilkörper in Form einer ringsum laufenden Dichtungswulst oder Dichtungsschneide 30, die im Ruhezustand einem Behälterwandabschnitt 32 anliegt, der den Ventilsitz bildet. Innerhalb des von der Dichtungswulst 30 umschlossenen Flächenabschnitts ist der Wandabschnitt 32 mit Durchtrittslöchern 34 versehen, die dem Medienaustausch dienen.

Die Kammerwand 28 ist durch eine innen liegende Platte 36 aus Metall oder Kunststoff versteift. Ebenso ist die gegenüberliegende Kammerwandung 38 durch eine Platte 40 versteift. Die zylindrische Seitenwand 42 zwischen der Vorderwand 28 und der Rückwand 38 ist derart flexibel, daß der Abstand zwischen Vorder- und Rückwand je nach den herrschenden Druckdifferenzen veränderbar ist. Diese Seitenwand kann balgartig oder harmonikaartig ausgebildet sein, und die Kammerwand kann einstückig hergestellt sein, oderaus mehreren Teilen bestehen, die dichtend miteinander verbunden sind. Die versteifungsplatten 36 und 40 dienen außerdem als Federteller für eine dazwischen ausgespannte Vorspann-Schraubenfeder 44, die die beiden Wände 28, 38 spreizt und das Kammervolumen zu vergrößern sucht. Die Gaskammer ist von einem mit Öffnungen versehenen Gehäuse 46 umschlossen, das an dem Wandabschnitt 32 angenietet oder auf andere Weise befestigt ist. Die Rückwand 38 kann mit dem Gehäuse 46 beispielsweise durch Vernietung verbunden sein.

Bei sämtlichen Ausführungsbeispielen wirkt der Gasdruck der Kammer unmittelbar auf die Ventilkörper ein. Dies hat den vorteil, daß die Ventile platzsparend an der Behälterwand angeordnet werden können. Falls dies in bestimmten Fällen als zweckmäßig erachtet werden sollte, könnte die Gasdruckkammer auch im Abstand zu den ventilkörpern angeordnet werden und es könnte die Abstützung über eine Hydraulikflüssigkeit erfolgen, die wiederum durch die Gasfeder, beispielsweise in einer Druckdose, abgestützt ist.

Zur Sterilisation des von einem Sterilisierbehälter aufgenommenen Sterilgutes in einem Dampf-Sterilisator sind, wie erwähnt, die folgenden Ventilfunktionen erforderlich:

1. Einströmventil: Durch dieses muß der der Sterilisation dienende Dampf in das Behälterinnere eingeleitet werden.

2. Vakuumventil: Durch dieses muß die ursprünglich im Behälter befindliche Luft und später der mit Restluft vermischte Dampf aus dem Behälter abgezogen werden.

3. Kondensatablaßventil: Durch dieses soll das Kondensat abgelassen werden, das sich innerhalb des Behälters gebildet hat, z. B. bei der Sterilisation von (nicht porösen) Instrumenten.

Die verschiedenen in ihrer Grundfunktion gleichen Ventile der dargestellten Ausführungsbeispiele können jeweils mehrere Funktionen durchführen. Daher wird im allgemeinen jedoch zweckmäßig sein, mehrere Ventile im Deckel bzw. im Boden anzubringen und diesen ventilen nur jeweils eine oder höchstens zwei Funktionen zuzuordnen. Im folgenden wird die Arbeitsweise der einzelnen Ventile hinsichtlich ihrer bevorzugten Anwendung beschrieben, was jedoch nicht ausschließt, daß eine Anwendung für einen anderen Zweck möglich ist.

Das Ventil gemäß Fig. 3 ist in erster Linie als Einströmventil geeignet und wird in dieser Funktion zweckmäßigerweise an dem als Deckel oder Zwischendeckel ausgebildeten Wandabschnitt 32 auf der Innenseite des Behälters aufgehängt.

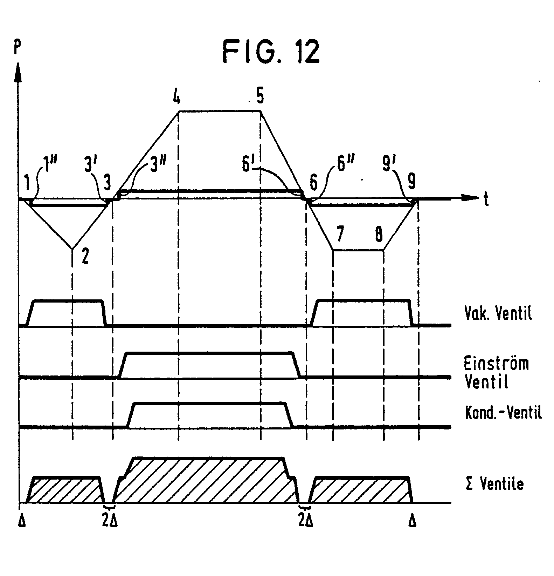

Vor Einbringen des Sterilisierbehälters in den Sterilisator wird das Ventil durch die Vorspannung der Feder 44 und gegebenenfalls durch eine Vorspannung der Gasdruckkammer 24 geschlossen. Die Gasdruckkammer ist mit Luft unter atmosphärischem Druck gefüllt und die atmosphärischen Luftdruckschwankungen spielen für die Funktion keine erhebliche Rolle. Beim anfänglichen einfachen oder mehrfachen Absaugen der Luft bzw. des Dampf-Restluftgehaltes in der Vakuum-Phase 1-2-3 (Fig. 12) bleibt das Ventil geschlossen, weil der Atmosphärendruck innerhalb der Kammer 24 positiv gegenüber dem Außendruck ist und infolge der Erwärmung durch den einströmenden Dampf noch verstärkt wird. In der nun folgenden Dampfeinströmphase 3-4 wird das Ventil geöffnet, und zwar an einer Stelle etwas über Atmosphärendruck, wegen der Vorspannung der Feder und gegebenenfalls wegen der Druckerhöhung durch Temperaturzunahme. Im Gegensatz zu den üblichen Rückschlagventilen schließt das erfindungsgemäße ventil jedoch nicht an der Stelle 4 maximalen Druckes, sondern bleibt über die gesamte Sterilisierphase 4-5 geöffnet, weil die Gaskammer des außen herrschenden Überdrucks wegen zusammengepreßt bleibt und der Ventilkörper abgehoben ist. Beim Druckabfall zwischen 5 und 6 (Fig. 12) bleibt das Ventil noch offen, bis an einer Stelle etwas über Atmosphärendruck (bedingt durch Federvorspannung und Lufterwärmung innerhalb der Kammer) das Ventil wieder schließt. In der nachfolgenden Vakuum-Phase 6-7-8-9 bleibt das Ventil geschlossen.

Fig. 4 zeigt ein erfindungsgemäßes Ventil in einer typischen Ausbildung und Anwendung als Vakuumventil. Hier ist die Kammerwandung 38 zusammen mit der Versteifungsplatte 40 an dem einen Teil des den Behälterdeckel bildenden Behälterabschnitts 32 festgelegt. Die in diesem Falle der gegenüberliegenden Kammerwand 28 außen anliegende Versteifungsplatte 36 greift mit einer Ringschneide 48 nach außen und ruht auf einem Dichtungsring 50, der in ein am Behälterdeckel festgelegtes U-Profil 52 eingesetzt ist. Die Vorspannfeder 44 stützt sich an der Platte 36 und dem Ventilgehäuse 46 ab.

Mit dem Buchstaben I ist in allen Figuren der Behälterinnenraum bezeichnet, und mit dem Buchstaben A der außerhalb des Sterilisierbehälters (im Autoklaven) befindliche Raum (dies gilt für die beschriebene bevorzugte Anwendung; bei abgewandelter Ausführung oder abgewandelter Nutzung ist eine Umkehrung denkbar).

Im Ruhezustand ist das Ventil gemäß Fig. 4 durch die vorspannung der Feder 44' geschlossen. Nach Ausgleich der Federvorspannung öffnet das Ventil 4 in der Vakuum-Phase kurz unterhalb der atmosphärischen Linie und bleibt während der gesamten Vakuum-Phase 1-2-3 offen, wobei die Einstellung bevorzugt so vorgenommen wird, daß die Öffnungsstellung auch erhalten bleibt, wenn Innenund Außendruck im Vakuumbereich gleich sind. Während der Überdruckphase 3-4-5-6, in der die Sterilisation stattfindet, bleibt das Ventil infolge des außen herrschenden Überdrucks geschlossen und öffnet wieder in der Vakuum-Phase 6-7-8-9.

Fig. 5 zeigt die Erfindung in der Anwendung bei einem typischen Kondensatablaßventil. Hier ist das Ventil an der Innenseite des vom Behälterboden 15 gebildeten Wandabschnitts 32 festgelegt. Das Kondensatablaßventil gemäß Fig. 5 verbleibt in der Öffnungsstellung, solange der Außendruck gegenüber dem Innendruck erhöht ist, d.h. während der gesamten Sterilisierphase 3-4-5-6 und nicht nur während der Einströmphase 2-3-4, wie dies bei einem Überdruckrückschlagventil der Fall ist. Gegenüber einem Bimetall-Ablaßventil besteht der Vorteil, daß das erfindungsgemäße Ventil unabhängig von den Temperaturbedingungen arbeitet sowie einen großen Hub und höheren Anpreßdruck besitzt.

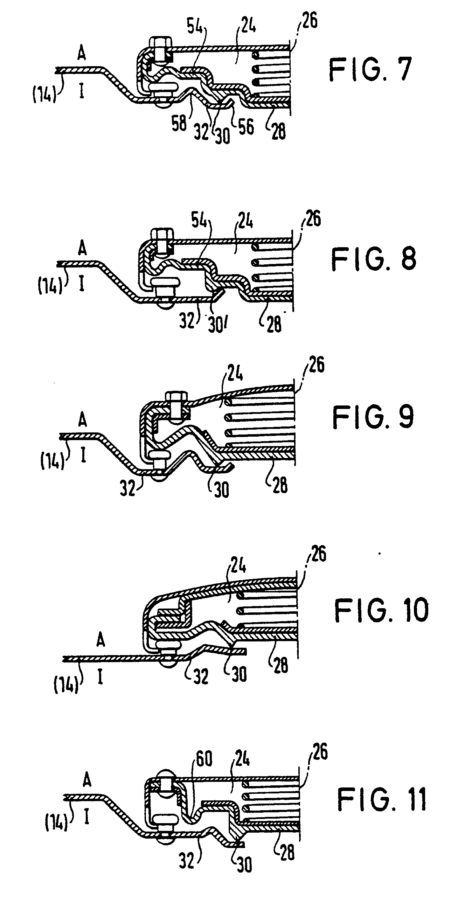

Die Bauart gemäß Fig. 6 ermöglicht eine Verwendung als Einströmventil, und in diesem Falle wird das Ventil außen auf dem Deckel 14 angebracht. Um ein Öffnen bei sich erhöhendem Außendruck zu gewährleisten, ist eine radial nach außen stehende Druckfläche 54 vorgesehen, mit der der Außendruck den Ventilkörper 30 abhebt, und nachdem ein Dichtungsspalt gebildet ist, kann der Außendruck an der Gesamtwandfläche 28 angreifen. Im übrigen ist die Funktion wie in Verbindung mit Fig. 3 beschrieben.

Wenn das Ventil gemäß Fig. 6 als Kondensatableiter benutzt werden soll, wird es in den Behälterboden 15 in der beschriebenen Weise eingesetzt und es arbeitet dann in gleicher Weise wie das in Verbindung mit Fig. 5 beschriebene Ventil.

Die Fig. 7 bis 11 veranschaulichen einige weitere abgewandelte Ausführungsbeispiele erfindungsgemäßer Ventile, die in der dargestellten Anordnung als Dampfeinströmventil arbeiten. Bei entsprechender Ausbildung ist jedoch auch eine Anwendung als Kondensat-Ablaßventil denkbar.

Bei dem Ventil 7 besteht die Besonderheit, daß die Behälterwand 32 mit einem nach innen hochstehenden Rand 56 versehen ist, den die Ventilkörperrippe 30 umschließt. Die außen liegende Druckfläche 54, die das anfängliche Abheben bewirkt, ist nicht wie bei dem Ausführungsbeispiel nach Fig. 6 abgeschrägt, sondern gestuft ausgebildet. Die Behälterwand ist außerdem radial außerhalb des Ventilkörpers 30 mit einer nach innen eingezogenen Rippe 58 versehen. Bei dem Ausführungsbeispiel nach Fig. 8 ist dieser letztgenannte Abschnitt eben ausgebildet und anstelle der Ventilkörperrippe ist eine Wulst 30' vorgesehen.

Bei den Ausführungsbeispielen nach Fig. 5 bis 9 bildet außerdem die Ventilgehäusewand gleichzeitig die äußere Begrenzung der Gaskammer 24.

Das Ausführungsbeispiel nach Fig. 10 unterscheidet sich von den übrigen Ausführungsbeispielen durch die Anordnung und Ausbildung der umlaufenden Ringwand. Bei dem Ausführungsbeispiel nach Fig. 11 erstreckt sich die Kammerwandung außerhalb der Ventilkörperrippe 30 in der Ebene des Deckels und senkrecht hierzu, wobei dazwischen eine elastische Sicke 60 ausgebildet ist.

Fig. 12 zeigt den zeitabhängigen Druckverlauf P innerhalb des Sterilisierbehälters und die Ventilfunktionen. Die stufenförmige Ausbildung der Kurven im Bereich des Nulldurchganges ist durch die Ventilvorspannung bedingt, d. h. durch den Federdruck und gegebenenfalls durch den Gasdruck innerhalb der Gasdruckkammer.

Der Sterilisiervorgang umfaßt die folgenden Phasen: ein-oder mehrfache Vakuumphase (1 bis 3), Überdruckphase (3 bis 6) mit Sterilisierphase (4 bis 5), Nachvakuumphase (5 bis 8), Belüftungsphase (8 bis 9). Das in jeder Phase wirksame Ventil wird im Gegensatz zu bekannten Vorrichtungen auch nach Erreichen des negativen oder positiven Spitzendruckwertes offengehalten, bis sich der Druck im Autoklaven auf einen vorbestimmten Schließschaltdruck (3', 6', 9') eingestellt hat. Außerdem wird das in jeder Phase wirksame ventil geschlossen gehalten, bis sich der Druck im Autoklaven auf einen vorbestimmten Öffnungsschaltdruck (1'', 3'', 6'') eingestellt hat. Dabei sind die Absolutwerte von Öffnungsschaltdruck und Schließschaltdruck zweckmäßig gleich groß.

Gemäß den dargestellten Ausführungsbeispielen werden die Ventile in Abhängigkeit von dem im Autoklaven herrschenden Druck gesteuert.

Stattdessen oder zusätzlich kann die ventilsteuerung auch in zeitlicher Abhängigkeit zu den Druck-Null-Durchgängen (atmosphärischer Druck) im Autoklaven oder in Abhängigkeit von der im Autoklaven herrschenden Temperatur erfolgen. Dabei kann jeder Ventilanordnung ein Sensor zugeordnet werden, der die Schaltfunktion auslöst. Schließlich ist es auch möglich, jede Ventilanordnung fremd zu steuern.

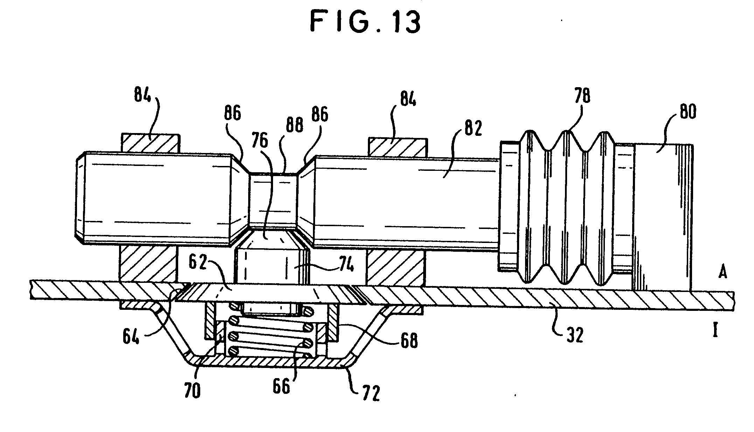

Fig. 13 zeigt ein Doppelventil, das als Einströmventil oder Vakuumventil, aber auch als Kondensatablaßventil verwendbar ist. Der ventilkörper 82, der mit einem Ventilsitz des Behälterwandabschnitts 32 zusammenwirkt, ist durch eine Feder 66 in die Schließstellung vorgespannt. Der Ventilkörper 62 ist mit einem zylindrischen Ansatz 68 auf einer Büchse 70 geführt, die über einen Halter 72 von der Behälterwand 32 getragen wird. Der Ventilkörper 62 trägt ein Betätigungsglied 74, welches einen kegelstumpfförmigen Kopf 76 aufweist. Die Gaskammer wird von einem Faltenbalg 78 umschlossen, der hermetisch abgedichtet und an einem Ende mit einem behälterfesten Klotz 80 verbunden ist. Das andere Ende des Faltenbalges 78 ist an einer Schubstange 82 festgelegt, die in Lagerböcken 84 axial verschiebbar geführt ist. Im Bereich des Betätigungsgliedes 74 weist die Schubstange 82 eine Eindrehung auf, die durch zwei konische Flächen 86 und einen dazwischenliegenden zylindrischen Abschnitt 88 begrenzt ist.

Fig. 13 zeigt eine Mittelstellung, in der das ventil geschlossen ist. Erhöht sich der Außendruck, dann wird der Balg 78 zusammengepreßt und die Schubstange 82 gemäß Fig. 13 nach rechts verschoben. Dadurch wird über die konischen Gleitflächen 86 und 76 das Ventil geöffnet.

Wenn demgegenüber ausgehend von der Mittelstellung gemäß Fig. 13 der Druck absinkt, dann dehnt sich der Faltenbalg 78 aus und die Schubstange 82 wird nach links verschoben, so daß das Ventil ebenfalls geöffnet wird.

Die axiale Länge des zylindrischen Abschnitts 88 ist etwas größer als der Durchmesser des Kopfendes 76, so daß ein gewisser Totgang eingebaut wird, der zur Folge hat, daß das Ventil innerhalb eines vorbestimmten Druckdifferenzbereichs geschlossen bleibt.

Demgemäß kann das in Fig. 13 dargestellte Ventil alle ventilfunktionen übernehmen, da es sowohl im Vakuumbereich als auch im Überdruckbereich durch den Faltenbalg 78 gesteuert wird.

Bei allen Ausführungsbeispielen kann eine Einstellung der Gaskammer auf den jeweiligen atmosphärischen Druck vorgenommen werden. Zu diesem Zweck kann ein in der Zeichnung nicht dargestelltes Ent- bzw. Belüftungsventil vorgesehen werden, das einen Druckausgleich ermöglicht. Im allgemeinen können jedoch äußere wetterbedingte Luftdruckschwankungen unberücksichtigt bleiben und es wird ausreichen, einen Druckausgleich auf die entsprechende Höhenlage vorzusehen, wo der Behälter benutzt werden soll. Zu diesem Zweck kann eine Öffung vorgesehen werden, die durch einen elastischen Stopfen dauerhaft abdichtbar ist.

Bei dem in Figur 13 dargestellten Ausführungsbeispiel ist die Betätigungsvorrichtung 78', 82', 86', 88 über die Lagerböcke 80, 84 an der Behälterwand festgelegt. Es soll für die Erfindung jedoch auch vorbehalten bleiben, diese Betätigungsvorrichtung vom Ventil zu trennen und lösbar so anzuordnen, daß diese Betätigungsvorrichtung nur im Sterilisator mit dem Ventil verbunden ist, so daß der Transport und die Lagerung des Sterilisierbehälters (außerhalb des Sterilisators) ohne diese Betätigungsvorrichtung erfolgen kann.

**Patentansprüche**

1. Verfahren zum Steuern der in einem Sterilisierbehälter für klinisches Sterilgut vorgesehenen Ventilanordnung während der aufeinanderfolgenden, im Inneren eines Autoklaven auftretenden Druckphasen: ein-oder mehrfache Vakuumphase (1 bis 3), Überdruckphase (3 bis 6) mit Sterilisierphase (4 bis 5) und vakuumphase (6 bis 9), dadurch gekennzeichnet, daß das in jeder Phase wirksame Ventil auch nach Erreichen des negativen oder positiven Spitzendruckwertes offengehalten wird, bis sich der Druck im Autoklaven auf einen vorbestimmten Schließschaltdruck (3', 6', 9') eingestellt hat.

2. Verfahren nach Anspruch 1, dadurch gekennzeichnet, daß das in jeder Phase wirksame ventil geschlossen gehalten wird, bis sich der Druck im Autoklaven auf einen vorbestimmten Öffnungsschaltdruck (1'', 3'', 6'') eingestellt hat.

3. Verfahren nach den Ansprüchen 1 und 2, dadurch gekennzeichnet, daß die Ventilsteuerung in Abhängigkeit von der im Autoklaven herrschenden Temperatur erfolgt.

4. Verfahren nach einem der Ansprüche 1 bis 3, dadurch gekennzeichnet, daß jeder Ventilanordnung ein Sensor zugeordnet wird, der die Schaltfunktion auslöst.

5. Ventil, welches gemäß einem der Ansprüche 1 bis 4 steuerbar und für Sterilisierbehälter bestimmt ist, die z. B. in einem Dampfsterilisator sterilisierbar sind, mit einem Ventilkörper, der in Abhängigkeit von einem durch den Sterilisator verursachten Druckdifferential öffnet und schließt, und der zusätzlich eine unter Druck deformierbare Kammer aufweist, dadurch gekennzeichnet, daß die ein vorbestimmtes Volumen unter vorbestimmtem Druck bei vorbestimmter Temperatur aufweisende Druckkammer (24) während des gesamten Sterilisiervorgangs (1 bis 9) gegenüber dem im Sterilisierbehälter herrschenden Innendruck und dem im Sterilisator herrschenden Außendruck abgedichtet ist.

6. Ventil nach Anspruch 5, dadurch gekennzeichnet, daß die Druckkammer (24) Luft unter atmosphärischem Druck enthält.

7. Ventil nach den Ansprüchen 5 und 6, dadurch gekennzeichnet, daß das Ventil eine Ringrippe (30) als Dichtungskörper aufweist, die mit einem Behälterwandabschnitt (32) als ventilsitz zusammenwirkt und Wanddurchgangslöcher (34) in diesem Wandabschnitt umschließt.

8. Ventil nach einem der Ansprüche 5 bis 7, dadurch gekennzeichnet, daß die Ventilkörperrippe (30) an einer Stirnwand (28) der Kammer ausgebildet ist, deren gegenüberliegende rückwärtige Stirnwand (38) am Ventilgehäuse (46) festgelegt ist, wobei der die beiden Stirnwände (28, 38) verbindende flexible umlaufende Wandabschnitt (42) elastisch faltbar ausgebildet ist, wobei die Stirnwände (28, 38) vorzugsweise durch Versteifungsplatten (36, 40) verstärkt sind und zwischen den Versteifungsplatten (36, 40) eine oder mehrere Vorspannschraubenfedern (44) und/oder ein Bimetall abgestützt sind.

9. Ventil nach einem der Ansprüche 5 bis 8, dadurch gekennzeichnet, daß die Gaskammerstirnwand einen nach außen über die Ventilkörperrippe (30) vorstehenden Druckrand (54) aufweist.

10. Ventil nach Anspruch 5, dadurch gekennzeichnet, daß der Ventilkörper von einer Ringschneide (48) gebildet ist, die von der Kammerwand (36) radial nach außen und axial nach innen vorsteht und mit einem Dichtungsring (50) zusammenwirkt, der am Behälterwandabschnitt (32) festgelegt ist.

11. Ventil nach Anspruch 10, dadurch gekennzeichnet, daß die Gaskammer (24) zwischen der die Dichtungsschneide (48) tragenden Wand (36) und der am Behälterdeckel (14) festgelegten Rückwand (38) angeordnet ist und daß die Vorspannfeder (44') zwischen der Rückwand (28) und der Ventilgehäusewand (46) angeordnet ist.

12. Ventil nach einem der Ansprüche 5 bis 11, dadurch gekennzeichnet, daß es mit eisnem eine Ausdehnungsflüssigkeit enthaltenden Gefäß verbunden ist.

13. Ventil nach einem der Ansprüche 5 bis 12, dadurch gekennzeichnet, daß ein oder mehrere Ventile in der Behälterwand derart angeordnet sind, daß nach Abschluß des Sterilisatiosnsverfahrens der Innenraum des Behälters unter Vakuum verbleibt.

14. Ventil nach einem der Ansprüche 5 bis 13, dadurch gekennzeichnet, daß es als Doppelventil ausgebildet ist und sowohl in der Überdruckphase als auch in der Vakuumphase öffnet.

15. Ventil nach Anspruch 14, dadurch gekennzeichnet, daß ein von der Gaskammer bewegbares Schaltglied das Ventil bei Bewegung aus seiner Mittelstellung in beide Schaltrichtungen öffnet.

**Revendications**

1. Procede pour commander l'ensemble des valves prévues dans un récipient de stérilisation pour produits stériles cliniques pendant les phases de pression successives intervenant à l'intérieur d'un autoclave: phase de vide simple ou multiple (1 à 3), phase de surpression (3 à 6) avec phase de stérilisation (4 à 5) et phase de vide (6 à 9), caractérisé par le fait que la valve, active dans chaque phase, est maintenue ouverte même après atteinte de la valeur de pression maximale négative ou positive jusqu'à ce que la pression dans l'autoclave se soit réglée à une pression de commutation de fermeture prédéterminée (3', 6', 9').

2. Procédé selon la revendication 1, caractérisé par le fait que la valve, active dans chaque phase, est maintenue fermée jusqu'à ce que la pression dans l'autoclave se soit réglée à une pression de commutation d'ouverture prédéterminée (1'', 3'', 6'').

3. Procédé selon les revendications 1 et 2, caractérisé par le fait que la commande de la valve s'effectue en fonction de la température régnant dans l'autoclave.

4. Procédé selon l'une des revendications 1 à 3, caractérisé par le fait qu'à chaque ensemble de valves est associé un capteur qui déclenche la fonction de commutation.

5. Valve pouvant être commandée selon l'une des revendications 1 à 4 et destinée à des récipients de stérilisation qui peuvent être stérilisés par exemple dans un stérilisateur à vapeur, comprenant un obturateur qui s'ouvre et se ferme en fonction d'un différentiel de pression provoqué par le stérilisateur, et qui comporte en plus une chambre déformable sous pression, caractérisée par le fait que la chambre à gaz (24), présentant un volume prédéterminé à une pression prédéterminée pour une température prédéterminée, est étanchée pendant tout le processus de stérilisation (1 à 9) vis-à-vis de la pression intérieure régnant dans le récipient de stérilisation et de la pression extérieure régnant dans le stérilisateur.

6. Valve selon la revendication 5, caractérisée par le fait que la chambre à gaz (24) contient de l'air à la pression atmosphérique.

7. Valve selon les revendications 5 et 6, caractérisée par le fait que la valve comporte une nervure annulaire (30) en tant qu'obturateur qui coopère avec une section de paroi (32) du récipient en tant que siège et qui entoure des trous de passage (34) pratiqués dans cette section de paroi.

8. Valve selon l'une des revendications 5 à 7, caractérisé par le fait que la nervure (30) de l'obturateur de la valve est réalisée sur une paroi frontale (28) de la chambre dont la paroi frontale arrière opposée (38) est fixée sur le corps (46) de la valve, la section de paroi périphérique flexible, raccordant les deux parois frontales (28, 38), étant réalisée élastique et pliable, les parois frontales (28, 38) étant de préférence renforcées par des plaques de renforcement (36, 40) et entre les plaques de renforcement (36, 40) étant insérés un ou plusieurs ressorts hélicoïdaux (44) et/ou un bilame.

9. Valve selon l'une des revendications 5 à 8, caractérisé par le fait que la paroi frontale de la chambre à gaz comporte un bord de pression (54) faisant saillie vers l'extérieur en dehors de la nervure (30) de l'obturateur de la valve.

10. Valve selon la revendication 5, caractérisée par le fait que l'obturateur est formé par un tranchant annulaire (48) qui, à partir de la paroi (36) de la chambre, fait saillie radialement vers l'extérieur et axialement vers l'intérieur et qui coopère avec un joint annulaire (50) qui est fixé sur la section de paroi (32) du récipient.

11. Valve selon la revendication 10, caractérisée par le fait que la chambre à gaz (24) est disposée entre la paroi (36) portant le tranchant d'étanchéité (48) et la paroi arrière (38) fixée au couvercle (14) du récipient et que le ressort (44') est inséré entre la paroi arrière (28) et la paroi (46) du corps de la valve.

12. Valve selon l'une des revendications 5 à 11, caractérisée par le fait qu'elle est raccordée à un vase contenant un liquide d'expansion.

13. Valve selon l'une des revendications 5 à 12, caractérisée par le fait qu'une ou plusieurs valves sont disposées dans la paroi du récipient de manière qu'à l'achèvement du processus de stérilisation l'espace interne du récipient reste sous vide.

14. Valve selon l'une des revendications 5 à 13, caractérisée par le fait qu'elle est réalisée sous la forme d'une valve cloche et s'ouvre aussi bien pendant la phase de surpression que pendant la phase de vide.

15. Valve selon la revendication 14, caractérisée par le fait qu'un organe de commutation, déplaçable par la chambre à gaz, ouvre la valve lorsque' à partir de sa position médiane, il se déplace dans les deux sens de commutation.

**Claims**

1. A method of controlling the valve arrangement provided in a sterilizing container for clinical sterile material during the successive pressure phases occurring inside an autoclave: single or multiple vacuum phase (1 to 3), overpressure phase (3 to 6) with sterilizing phase (4 to 5) and vacuum phase (6 to 9), characterized in that the valve effective in each phase is kept open even after the negative or positive peak pressure value is reached, until the pressure in the autoclave has adjusted itself to a predetermined closure switching pressure (3', 6', 9').

2. A method according to claim 1, characterized in that the valve effective in each phase is kept closed until the pressure in the autoclave has adjusted itself to a predetermined opening switching pressure (1'', 3'', 6'').

3. A method according to claims 1 and 2, characterized in that the valve control is effected in dependence upon the temperature obtaining in the autoclave.

4. A method according to any one of claims 1 to 3, characterized in that a sensor initiating the switching function is associated with each valve arrangement.

5. A valve which is controllable according to any one of claims 1 to 4 and intended for sterilizing containers which are sterilizable for example in a steam sterilizer, comprising a valve body which opens and closes in dependence upon a pressure differential caused by the sterilizer and which additionally comprises a chamber deformable under pressure, characterized in that the pressure chamber (24) having a predetermined volume under predetermined pressure and at predetermined temperature is sealed during the entire sterilizing process (1 to 9) with respect to the internal pressure obtaining in the sterilizing container and the external pressure obtaining in the sterilizer.

6. A valve according to claim 5, characterized in that the pressure chamber (24) contains air under atmospheric pressure.

7. A valve according to claims 5 and 6, characterized in that the valve comprises an annular rib (30) as a sealing body, which cooperates with a container wall portion (32) as a valve seat and surrounds wall through-holes (34) in this wall portion.

8. A valve according to any one of claims 5 to 7, characterized in that the valve body rib (30) is formed at one end wall (28) of the chamber, the opposite rear end wall (38) of which is secured to the valve housing (46), the flexible surrounding wall portion (42) connecting the two end walls (28, 38) being made elastically foldable, the end walls (28, 38) preferably being reinforced by stiffening plates (36, 40) and one or more biasing helical springs (44) and/or a bimetal element being supported between the stiffening plates (36, 40).

9. A valve according to any one of claims 5 to 8, characterized in that the gas chamber end wall comprises a pressure edge (54) projecting outwards beyond the valve body rib (30).

10. A valve according to claim 5, characterized in that the valve body is formed by an annular edge (48), which projects radially outwards and axially inwards from the chamber wall (36) and cooperates with a sealing ring (50) which is secured to the container wall portion (32).

II. A valve according to claim 10, characterized in that the gas chamber (24) is arranged between the wall (36) carrying the sealing edge (48) and the rear wall (38) secured to the container lid (14) and in that the biasing spring (44') is arranged between the rear wall (28) and the valve housing wall (46).

12. A valve according to any one of claims 5 to 11, characterized in that it is connected to a vessel containing an expansion fluid.

13. A valve according to any one of claims 5 to 12, characterized in that one or more valves are arranged in the container wall in such a way that after conclusion of the sterilization process the inside of the container remains under vacuum.

14. A valve according to any one of claims 5 to 13, characterized in that it is constructed as a double valve and opens both in the overpressure phase and in the vacuum phase.

15. A valve according to claim 14, characterized in that a switching member movable by the gas chamber opens the valve on movement out of its central position in both switching directions.

# FIG. 1

FIG. 2

# FIG. 3

# FIG. 4

# FIG. 5

# FIG. 6

3

FIG. 7

FIG. 8

FIG. 9

FIG. 10

FIG. 11

4

# FIG. 12

Vak. Ventil

Einström Ventil

Kond.-Ventil

Σ Ventile

# FIG.13

EP 0 267 935 B1